# EUROPÄISCHE PATENTANMELDUNG

(11) **EP 1 321 104 A1**
(43) Veröffentlichungstag der Anmeldung: **25.06.2003**
(21) Anmeldenummer: 01129004.6
(22) Anmeldetag: 06.12.2001
(51) Int. Cl.: A61B 18/08, A61F 9/007

(54) **Ablationsinstrument und Verfahren zum Schneiden, Fragmentieren und/oder Abtragen von Material**

(71) Anmelder: GFD-Gesellschaft für Diamantprodukte mbH, 89081 Ulm (DE)
(72) Erfinder: Gluche, Peter, Dr., 89073 Ulm (DE); Ertl, Stephan, 89075 Ulm (DE); Lingenfelder, Christian, 89075 Ulm-Mähringen (DE); Kohn, Erhard, Prof. Dr., 89081 Ulm/Lehr (DE)
(74) Vertreter: Pfenning, Meinig & Partner

(57) **Zusammenfassung**

Die Erfindung betrifft ein Ablationsinstrument (2) sowie ein Verfahren zum Schneiden, Fragmentieren und/oder Abtragen von Material (15). Derartige Verfahren werden benötigt, um von einem Gegenstand Material (15) abzutragen bzw. diesen einzuschneiden oder zu zerstören. Insbesondere, jedoch nicht ausschließlich, werden derartige Verfahren und Ablationsinstrumente im Bereich der Medizin, insbesondere im Bereich der Katarakteextraktion minimalinvasiven Chirurgie oder der Vitrektomie benötigt.

## Beschreibung

Die vorliegende Erfindung betrifft ein Ablationsinstrument sowie ein Verfahren zum Schneiden, Fragmentieren und/oder Abtragen von Material. Derartige Verfahren werden benötigt, um von einem Gegenstand Material abzutragen bzw. diesen einzuschneiden oder zu zerstören. Insbesondere, jedoch nicht ausschließlich, werden derartige Verfahren und Ablationsinstrumente im Bereich der Medizin, insbesondere im Bereich der Katarakteextraktion, minimalinvasiven Chirurgie oder der Vitrektomie benötigt.

Nach dem Stand der Technik werden zur Zerstörung der Augenlinse Ultraschallschneidelemente verwendet. Hierbei wird mittels eines Piezokristalls eine scharfe Schneide sehr rasch hin und her bewegt und dadurch das Material der Linse zerstückelt. Alternativ wird über einen Applikatorstab ein Target in die Linse eingeführt, auf das ein Laserstrahl gerichtet wird. Der Laserstrahl erhitzt nun das Target und dieses erzeugt eine Dampfblase in dem Material, die das Material aufgrund ihrer explosionsartigen Expansion im Umkreis der Blase zerstört. Dieses zerstörte fragmentierte Material kann dann beispielsweise durch das Applikatorröhrchen abgesaugt werden. Als Target werden dabei Titan oder Tantal verwendet, wobei durch Einstrahlung eines geeigneten Laserpulses kurzfristige Hotspots mit einer Temperatur von über 320°C erreicht werden.

Diese herkömmlichen Systeme sind nicht zuletzt aufgrund des benötigten Lasersystems komplex und sehr teuer. Weiterhin sind die Applikatorstäbe komplex aufgebaut und kompliziert zu handhaben.

Aufgabe der vorliegenden Erfindung ist es daher, ein Ablationsinstrument und ein Verfahren zum Schneiden, Fragmentieren und/oder Abtragen von Material eines Gegenstandes zur Verfügung zu stellen, das auf einfache, präzise und kostengünstige Weise eine derartige Materialbehandlung ermöglicht.

Diese Aufgabe wird durch das Ablationsinstrument gemäß Anspruch 1 und das Verfahren nach Anspruch 38 gelöst. Vorteilhafte Weiterbildungen des erfindungsgemäßen Instrumentes und des erfindungsgemäßen Verfahrens werden in den jeweiligen abhängigen Ansprüchen gegeben.

Erfindungsgemäß weist das Ablationsinstrument mindestens eine Widerstandsheizschicht aus dotiertem oder graphitischen Diamant auf. Diese Heizschicht wird nun über elektrische Zuleitungen und Ableitungen mit einem Strom beaufschlagt und dient zur Erzeugung sehr hoher Temperaturen. Die Heizschicht ist dabei auf einem Substrat angeordnet, so daß sie, beispielsweise über eine Halterung, in unmittelbaren Kontakt mit dem zu behandelnden Gegenstand oder in dessen Nähe gebracht werden kann. Sofern das Material nicht unmittelbar kontaktiert wird, kann zusätzlich zwischen die Heizschicht und das Material eine Flüssigkeit eingebracht werden. Die Heizschicht und damit die mit dieser kontaktierte Flüssigkeit oder das mit der Heizschicht kontaktierte Material werden nun durch kurze Strompulse temporär auf sehr hohe Temperaturen aufgeheizt, so daß sich Dampfblasen bilden, die wie auch im Stand der Technik zu einer explosionsartigen Absprengung oder Fragmentierung des Materials führen.

Für das erfindungsgemäße Ablationsinstrument eignet sich eine Widerstandsheizschicht aus dotiertem oder graphitischem Diamant insbesondere aufgrund dessen hervorragenden hierfür geeigneten Eigenschaften. Diese sind insbesondere seine elektrische Leitfähigkeit, seine sehr hohe thermische Stabilität, die auch bei hohen Temperaturen nicht zu einer Zerstörung des Ablationsinstrumentes führt, sowie die hohe Wärmeleitfähigkeit von Diamant, die dazu führt, daß die Widerstandsheizschicht sehr kurzzeitig und überaus homogen aufgeheizt werden kann. Zu erwähnen ist ferner die geringe Wärmekapazität die eine schnelle Energieabgabe ermöglicht.

Vorteilhafterweise ist zwischen dem Substrat und der Widerstandsheizschicht eine thermisch isolierende Schicht angeordnet, um eine Ableitung der Wärme in das Substrat zu behindern und so die Aufheizbarkeit der Widerstandsheizschicht zu verbessern und die mögliche Frequenz der einzelnen Wärmepulse zu erhöhen.

Weiterhin kann zwischen der thermisch isolierenden Schicht und der Widerstandsheizschicht eine oder mehrere Zwischenschichten, insbesondere aus intrinzischem Diamant, angeordnet sein, um beispielsweise die bei hohen Temperaturen durch ein thermisch isolierendes Substrat, beispielsweise aus SiO₂, durchgehenden Leckströme, zu unterbinden und außerdem die Bekeimungsfähigkeit der thermisch isolierenden Schicht mit dotiertem oder graphitischem Diamant zu verbessern. Weiterhin kann die Widerstandsheizschicht auf ihrer dem zu behandelnden Material zugewandten anderen Seite eine elektrisch isolierende Schicht aufweisen, die dazu führt, daß der Strom tatsächlich ausschließlich auf die Widerstandsheizschicht beschränkt wird.

Ganz wesentlich bei der vorliegenden Erfindung ist weiterhin, daß Diamant als Oberfläche chemisch und physikalisch inert ist und daher sich insbesondere für medizinische, beispielsweise intrakorporale, Anwendungen eignet.

Erfindungsgemäß kann die Widerstandsheizschicht mit einzelnen Strompulsen mit Frequenzen bis ca. 50 kHz bei Kontaktierung mit Wasser beaufschlagt werden. Diese Strompulse können periodisch oder aperiodisch wiederholt werden. Die Grenzfrequenz für die Strompulse ist dabei selbstverständlich abhängig vom Umgebungsmedium, insbesondere dessen Viskosität und thermische Eigenschaften.

Vorteilhaft an der vorliegenden Erfindung ist, daß keine komplizierten optischen Bauelemente (Laser) oder mechanisch bewegte Teile (Klinge mit Piezoantrieb) verwendet werden. Damit ist auch die Wahrscheinlichkeit für ein Versagen des Ablationsinstrumentes weitgehend ausgeschlossen. Bei dem erfindungsgemäßen Ablationsinstrument sind Leistungsdichten von über 700 kW pro cm² bzw. sehr kleine Flächen der Widerstandsheizschicht von beispielsweise 0,5 mm² möglich, da Diamant eine extrem hohe mechanische Stabilität, elektrische Leitfähigkeit, thermische Leitfähigkeit und einen geringen thermischen Expansionskoeffizienten aufweist. Aufgrund der guten thermischen Leitfähigkeit und der homogenen Verteilung können auch Heizelemente > 1 mm², bevorzugt 1 bis 10 mm², ganz besonders bevorzugt 1 bis 5 mm² realisiert werden, mit denen große Blasen erzeugt werden können. Dies ermöglicht eine unmittelbare Umwandlung elektrischer in Heizenergie, eine sehr homogene Wärmeverteilung auf dem Heizelement, keine Kavitationsschäden am Heizelement aufgrund der erzeugte Blase sowie keinen thermischen Streß für das Heizelement durch die extrem rasche Aufheizung und Abkühlung. Insgesamt ist damit ein sehr zuverlässiges Ablationsinstrument gegeben, das aufgrund seiner Dimensionierung und Kosten selbst als Einweginstrument verwendet werden kann.

Wird in das Ablationsinstrument, beispielsweise unmittelbar in die Heizschicht, ein Thermoelement mit integriert, so ist auch eine Steuerung der erzeugten Temperatur, beispielsweise in Abhängigkeit vom Umgebungsmedium, möglich. Das Heizelement selbst kann dabei das Thermoelement darstellen.

Erfindungsgemäß kann durch eine vorgewählte Gestaltung der Widerstandsheizschicht die Form der erzeugten Blase vorgewählt werden. Auch die Temperatur, bei der die Blase erzeugt wird, kann bestimmt werden, beispielsweise als nahe oder oberhalb der Spinodaltemperatur.

Bei einem Ablationsinstrument, das mindestens eine Widerstandsschicht aus dotiertem oder graphitischem Diamant (diamond-like carbon) enthält, die auf einer thermisch isolierenden Schicht angeordnet ist, ist die Diffusion der in der Widerstandsschicht generierten Wärme deutlich reduziert und der Wirkungsgrad der Heizschicht somit erheblich verbessert. Im Gegensatz zu Heizschichten des Standes der Technik befindet sich die erfindungsgemäße Heizschicht aufgrund entsprechender Strukturierung weder in thermischem Kontakt zu einem großflächigen Diamantfilm noch zu anderen Wärmesenken. Eine laterale Diffusion der Wärme läßt sich durch Maßnahmen wie Mesaätzen oder selektives Wachstum verhindern. Aufgrund der eine thermische Isolationsschicht aufweisenden vertikalen Schichtstruktur wird auch eine Wärmediffusion in das Substrat ausgeschlossen. Die Verwendung der Isolationsschicht macht das aus dem Stand der Technik bekannte Dünnen des Substrates überflüssig, so daß die erfindungsgemäßen Heizschicht auf mechanisch stabilen, kommerziellen Substraten angeordnet werden können. Die isolierende Schicht weist bevorzugt eine Wärmeleitfähigkeit von unter 1 W cm⁻¹ K⁻¹ bei 300 K auf und kann aus Materialien wie beispielsweise Siliziumoxiden, Siliziumnitriden, Siliziumoxinitriden und Aluminiumoxiden bestehen. Um eine ausreichende thermische Isolierung der Heizschicht zu gewährleisten, sollte die Dicke der isolierenden Schicht mindestens 0,25 µm betragen und bevorzugt in einem Bereich zwischen 1 µm und 10 µm liegen. Besonders bevorzugt besteht die Isolationsschicht aus amorphen Materialien, welche geringe thermische Leitfähigkeiten besitzen. Die Isolationsschicht weist bevorzugt keine oder eine nur geringe elektrische Leitfähigkeit auf.

Die dotierte Widerstandsheizschicht kann direkt auf der isolierenden Schicht gewachsen werden. Alternativ ist es auch denkbar, zwischen Widerstandsheizschicht und isolierender Schicht eine oder mehrere Zwischenschichten vorzusehen. Um eine laterale Wärmediffusion zu vermeiden, sollte die mindestens eine Zwischenschicht entweder selbst ein thermischer Isolator sein oder aber lateral strukturiert werden.

Die Widerstandsheizschicht des Ablationsinstrumentes besitzt bevorzugt eine Fläche von weniger als 1 mm² und besonders bevorzugt von weniger 100 x 100 µm².

Wie bereits ausgeführt, können auch Flächen von 1 bis 10 mm², bevorzugt 1 bis 5 mm² realisiert werden, mit denen eine Blase erzeugt werden kann.

Eine bevorzugte Ausgestaltung der Erfindung sieht vor, die Wärmeabgabe des erfindungsgemäßen Ablationsinstrumentes durch mit der Heizschicht monolithisch integrierte Temperatursensoren zu erfassen, um eine exakte Steuerung des Ablationsinstrumentes zu gewährleisten. Die Dotierstoffkonzentration in der Widerstandsschicht sollte zur Erzeugung eines temperaturunabhängigen elektrischen Widerstandes daher mindestens etwa 10¹⁹. cm⁻³ betragen. Bei geringerer Dotierstoffkonzentration und wenn eine gewisse Temperaturabhängigkeit des Heizverhaltens in Kauf genommen wird, kann das Ablationsinstrument auch selbst als Temperatursensor fungieren.

Im folgenden werden einige Beispiele erfindungsgemä- ßer Ablationsinstrumente und erfindungsgemäßer Verfahren gegeben.

### Es zeigen:

- Fig. 1: ein herkömmliches Ablationsinstrument;
- Fig. 2: den Aufbau eines erfindungsgemäßen Ablationsinstrumentes;
- Fig. 3: verschiedene weitere Ablationsinstrumente;
- Fig. 4: eine Aufsicht auf ein Ablationsinstrument;
- Fig. 5: den Verlauf der Blasenerzeugung mit einem erfindungsgemäßen Ablationsinstrument;
- Fig. 6 bis Fig. 13: verschiedene Formen erfindungsgemäßer Ablationsinstrumente.

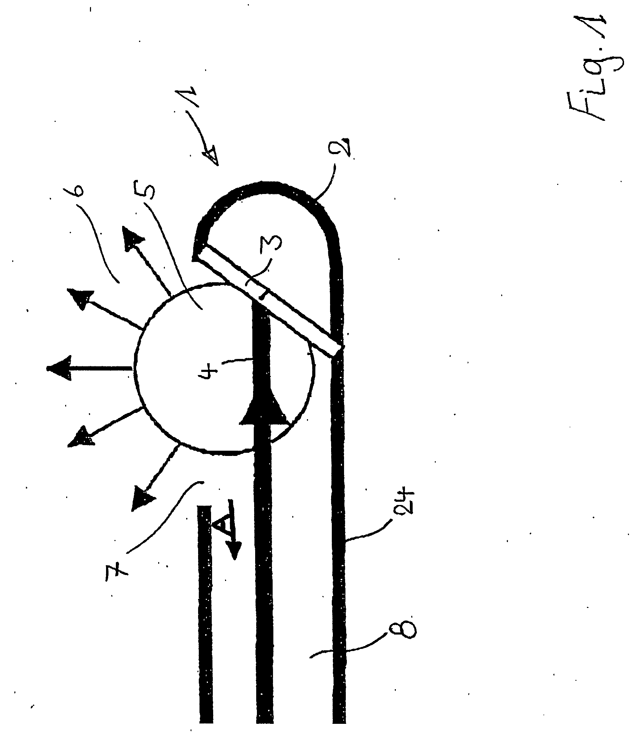

Fig. 1 zeigt ein Ablationsinstrument 1 nach dem Stand der Technik. Dieses Ablationsinstrument besteht aus einem Applikatorstab in Form eines Röhrchens 2, das an dem in Fig. 1 dargestellten Ende eine seitliche Öffnung 7 aufweist. Weiterhin ist unter einem Winkel zu der axialen Richtung des Röhrchens 2 ein Target 3 angeordnet, das beispielsweise aus Titan oder Tantal besteht. Dieses Röhrchen 2 besitzt eine kanalartige Durchgangsöffnung 8, die durch die Außenwände 24 des Röhrchens 2 gebildet wird und mit der Öffnung 7 kommuniziert.

Wie in Fig. 1 dargestellt, wird nun zur Materialzerstörung ein Laserstrahl 4 auf das Target 3 gegeben. Das Target 3 steht mit einer Flüssigkeit oder dem umgebenden Gewebe, beispielsweise einer Linse, in Kontakt. Durch den Laserstrahl 4 wird das Target 3 auf eine sehr hohe Temperatur aufgeheizt, gewöhnlich bis zu 321°C. Hierdurch wird das mit dem Target 3 in Kontakt befindliche Medium 6 überhitzt und bildet eine Gasblase 5, die sich explosionsartig in Richtung der dargestellten fünf Pfeile ausdehnen. Diese Gasblase überträgt eine Schockwelle auf das Medium 6 und zerstört dieses dadurch. Anschließend wird das abgetragene Material in Richtung des Pfeiles A durch die Öffnung 8 abgesaugt.

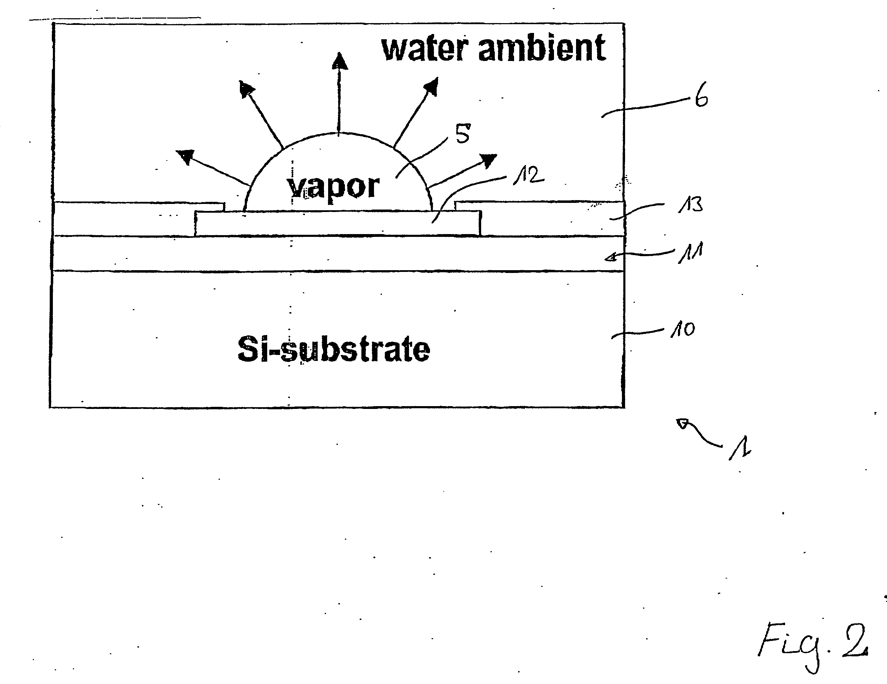

Fig. 2 zeigt die Struktur eines erfindungsgemäßen Ablationswerkzeuges 1. Dieses weist ein Siliziumsubstrat 10 auf, auf dem eine thermisch isolierende Schicht 11 sowie eine Widerstandsheizschicht 12 aufgebracht ist. Die Widerstandsheizschicht 12 ist über elektrische Kontakte 13 (Metallisierungen) kontakiert. Wiederum steht die obere Fläche der Widerstandheizschicht 12 aus Diamant mit dem Medium 6 in Kontakt. Wird nun ein Strom über die Metallisierung 13 durch die Diamantschicht 12 geschickt, so heizt sich die Diamantschicht 12 auf und bildet wiederum eine Gasblase 5 in dem Medium 6 aus.

Vorteilhaft bei dem erfindungsgemäßen Ablationsinstrument ist weiterhin aufgrund der Inertheit des aktiven Elementes die gute Reinigungsmöglichkeit, da kaum oder gar kein Gewebe anhaftet.

Zur Herstellung der in Fig. 2 dargestellten Schichtstruktur wird auf einem Siliziumcarbid oder Siliziumsubstrat 10 zunächst mit Hilfe der CVD-Technik eine 3 µm dicke SiO₂-Schicht 11 abgeschieden. Ebenfalls mittels eines CVD-Verfahrens wird auf der SiO₂-Schicht 11 eine P⁺-dotierte Diamantschicht mit einer Dotierstoffkonzentration von 10²⁰ bis 10²¹ cm⁻³ und einer Dicke von 400 nm bis 10 µm, bevorzugt 1 bis 3 µm aufgewachsen. Der P⁺-dotierte Diamantfilm fungiert als Widerstandheizschicht 12.

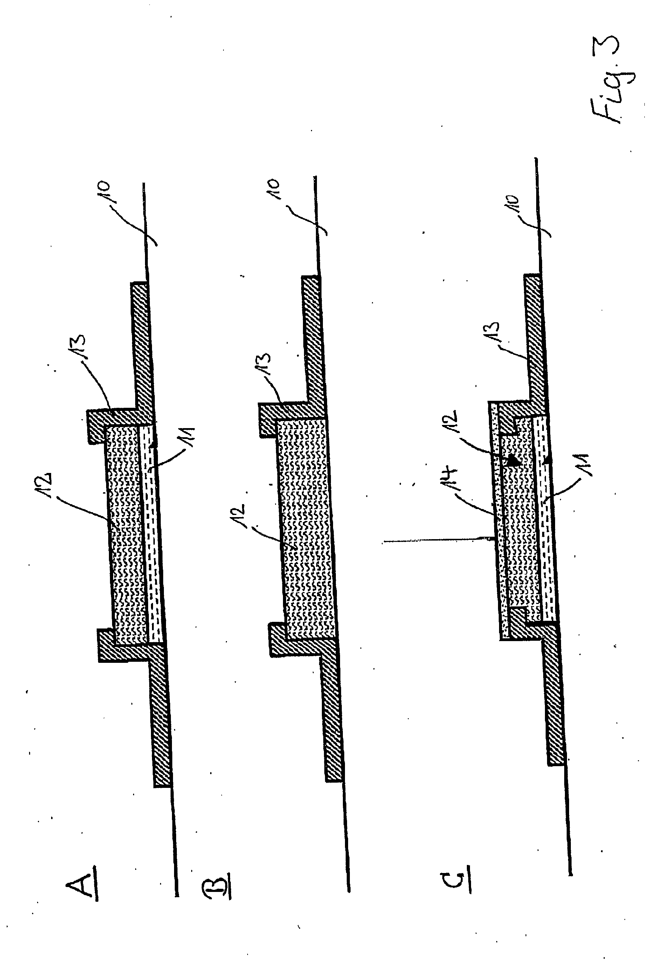

Fig. 3 zeigt in den Teilfiguren A-C den Aufbau dreier verschiedener erfindungsgemäßer Ablationswerkzeuge.

In Fig. 3A wird auf einem Substrat 10 eine thermische Isolationsschicht 11 aus SiO₂ aufgebracht und auf dieser eine Widerstandsheizschicht 12 aus dotiertem Diamant. Die metallischen Kontaktierungen 13 erstrekken sich von der Widerstandsheizschicht 12 über das Substrat 10.

In Fig. 3B befindet sich die Widerstandsheizschicht 12 unmittelbar auf dem Substrat und in Fig. 3C ist auf Widerstandsheizschicht 12 und auch die niveaugleichen Teile der metallischen Kontaktieren 13 eine elektrisch isolierende Schicht 14 aus intrinsischem Diamant oder SiO₂ aufgebracht. Diese elektrische Isolationsschicht 14 besitzt nur eine geringe Schichtdicke, damit eine Erhitzung des Mediums dennoch zuverlässig gewährleistet ist.

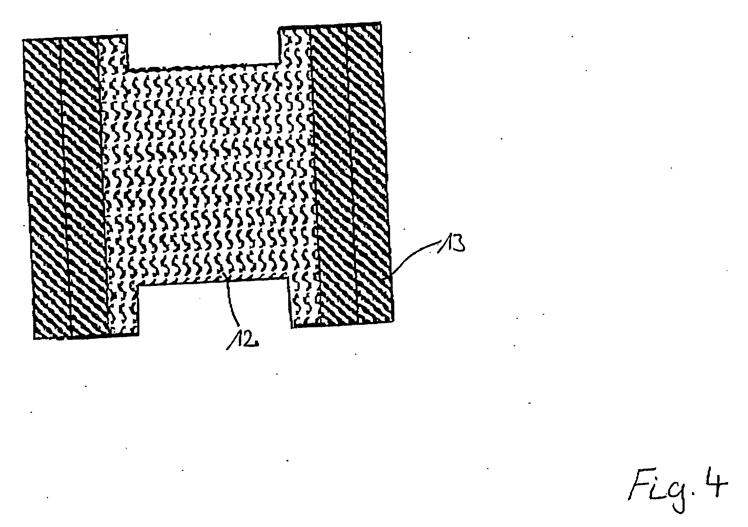

Als Materialien kommen hierbei elektrische Isolatoren mit guter Wärmeleitfähigkeit (z.B. Diamant) in Frage. Fig. 4 zeigt eine Aufsicht auf die Widerstandsheizschicht 12 und die elektrische Kontaktierung 13 für Ablationswerkzeuge wie in Fig. 3A und 3B dargestellt. Hier ist zu erkennen, daß die Widerstandsheizschicht 12 zwischen den beiden Kontaktschichten eingeschnürt ist und verglichen mit der geringsten Breite der Widerstandsheizschicht 12 die Kontaktflächen zwischen der Metallisierung 13 und der Widerstandsschicht 12 sehr breit und großflächig sind. Dadurch werden die Übergangswiderstände zwischen der Widerstandsschicht 12 und den Kontaktierungen 13 herabgesetzt, so daß im wesentlichen die Wärmeerzeugung in der Widerstandsheizschicht 12 erfolgt.

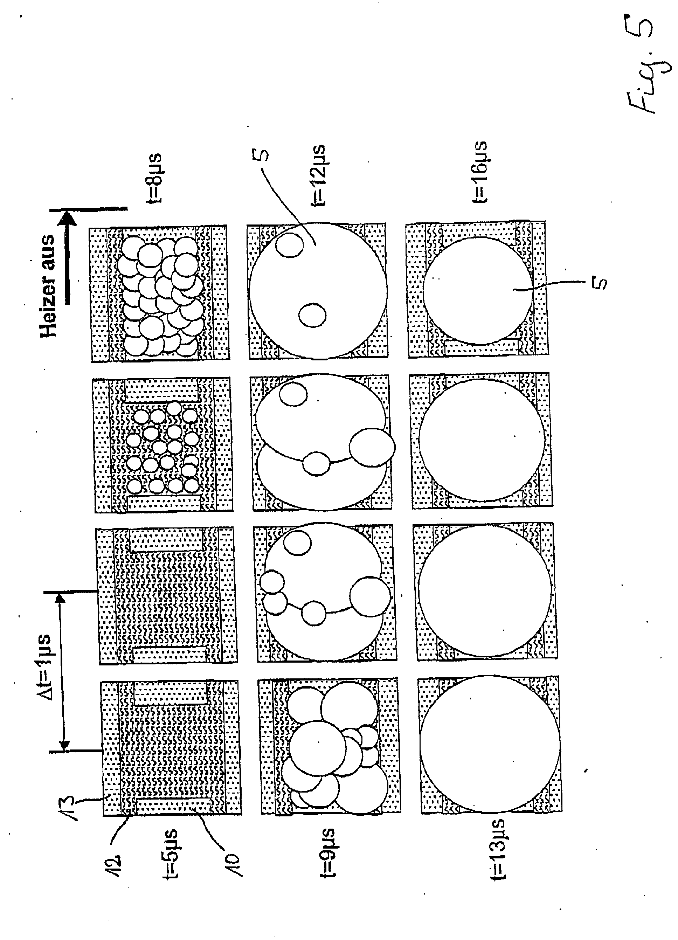

Fig. 5 zeigt die Erzeugung einer Gas- bzw. Dampfblase in Wasser bei einer elektrischen Leistung von 10 W in der zeitlichen Abfolge, wobei die einzelnen Bilder von links nach rechts und oben nach unten aufeinanderfolgen. Die zeitlichen Abstände zwischen den einzelnen Bildern betragen 1 µsec, wobei das erste Bild links oben zu einem Zeitpunkt t = 5 µsec nach dem Beginn des Stromflusses durch den Heizer beginnt und das letzte Bild recht unten den Zeitpunkt bei t = 16 µsec angibt. Bei diesem Beispiel wurden insgesamt für 8 µsec ein elektrischer Strom auf das Widerstandsheizelement 12 gegeben.

Wie in Fig 5 zu erkennen ist, bilden sich bereits nach 7 µsec kleine lokale Gasblasen, die sich bis nach 12 µsec zu einer großen Gasblase vereinigen, die ihren größten Ausdehnungszustand nach 13 µsec ausbildet. Danach bildet sich die Gasblase 5 wieder zurück.

Diese rasche innerhalb von µ-Sekunden erfolgende Ausbildung und Rückbildung der Gasblasen ermöglicht eine Frequenz der Gasblasenbildung zwischen 0 und 30.000 Hertz bei einer Energie pro Puls von 0-7 mJ. Dies entspricht Leistungen von 0-145 W. Es wurden daher bereits Leistungsdichten von 0-210 kW/cm² bei einer Oberfläche des Heizelementes von 60 x 60 µm², einer Strompulsdauer von 7 µsec und einer Frequenz von 20 kHz gemessen.

Damit liegt das erfindungsgemäße Ablationsinstrument bezüglich der Frequenz, der Energie pro Puls und der in das umgebende Medium eingebrachten Leistung sowie der Leistungsdichte weiter oberhalb der herkömmlichen Ultraschall- oder Laserverfahren.

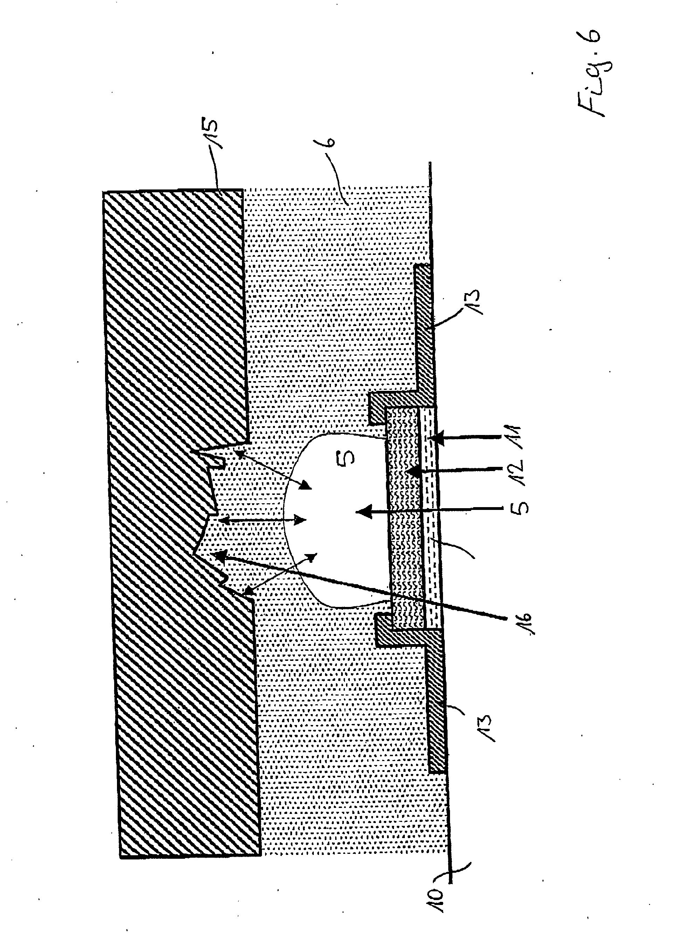

Fig. 6 zeigt den Einsatz eines Ablationswerkzeuges gemäß. Fig. 3a. Im Einsatz zum Materialabtrag von einem Gegenstand 15. In diesem Falle wird zwischen dem Gegenstand 15 und die Widerstandsheizschicht 12 ein Medium 6, beispielsweise Wasser, eingebracht. Dieses bildet nun die erfindungsgemäßen Blasen aus und zerstört durch die kavitative Wirkung dieser Blase die Oberfläche des Gegenstandes 15. Dadurch wird die Oberfläche des Gegenstandes 15 fragmentiert und abgetragen. Die erzeugten Bruchstücke können dann anschließend abgesaugt oder anderweitig entfernt werden.

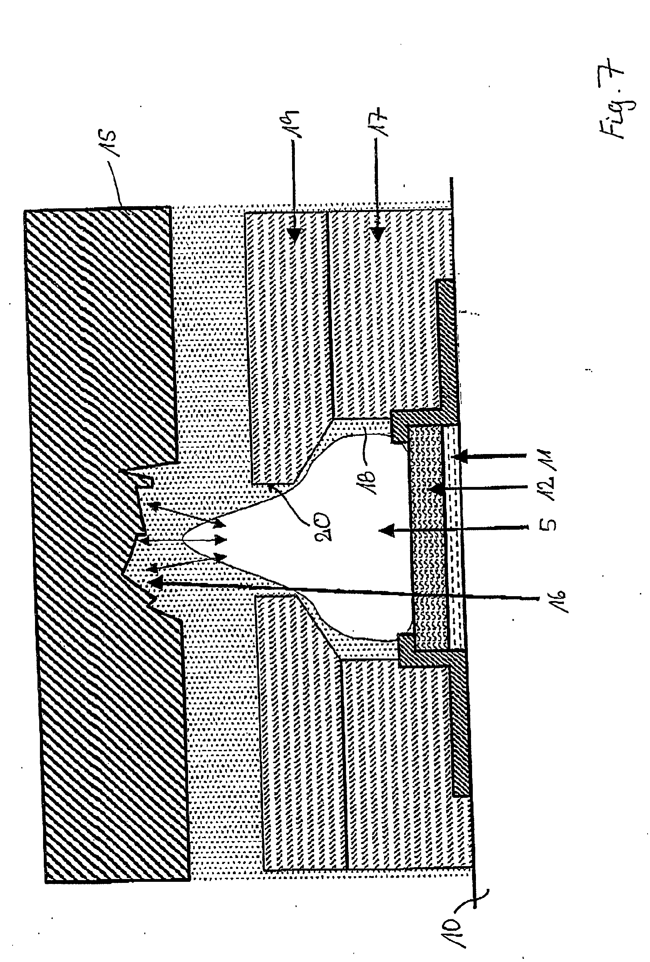

Fig. 7 zeigt ein Ablationswerkzeug, das einen ähnlichen Aufbau wie in Fig. 3A aufweist. Zusätzlich ist oberhalb der Widerstandsheizschicht 12 ein Kapillarsystem 17 angeordnet, das hier in senkrechter Richtung zur Zeichnungsebene über eine Kapillare 18 ein Medium auf die Oberfläche des Heizelementes 12 transportiert.

Oberhalb des Kapillarsystems 17 ist weiterhin eine Düsenplatte angeordnet, durch die der Querschnitt der Kapillare weiter verengt wird und so eine schmale Düsenöffnung gebildet wird, die die Kapillare 18 und die Oberfläche der Widerstandsheizschicht 12 mit der Außenseite des Ablationswerkzeuges verbindet. Bei dieser Anordnung wird die erzeugte Gasblase 5 durch die in der Düsenplatte 19 gebildete Düsenöffnung 20 ausgeschleudert, wodurch die kavitative Wirkung auf den Gegenstand 15 noch verstärkt werden kann. Mittels des Kapillarsystems ist es weiterhin möglich, immer ausreichendes zu verdampfendes Medium der Widerstandsheizschicht 12 zuzuführen.

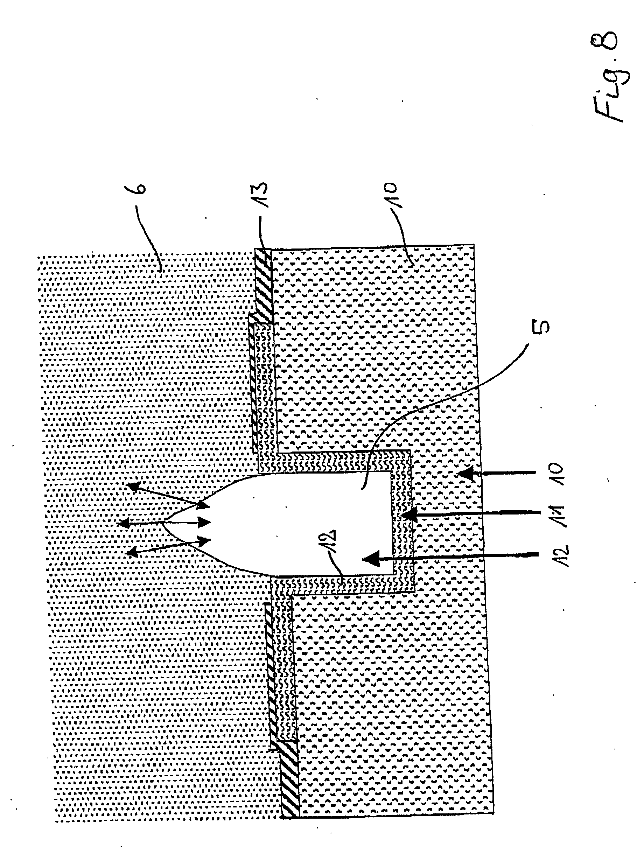

Fig. 8 zeigt ein weiteres erfindungsgemäßes Ablationswerkzeug, bei dem die Widerstandsheizschicht 12 dreidimensional geformt ist und in dem Substrat 10 einen Topf bildet. Dadurch die Kontaktoberfläche der Widerstandsheizschicht 12 mit dem Medium 6 vergrößert und die Widerheizschicht 12 bildet selbst eine düsenartige Öffnung an ihrer Oberseite aus.

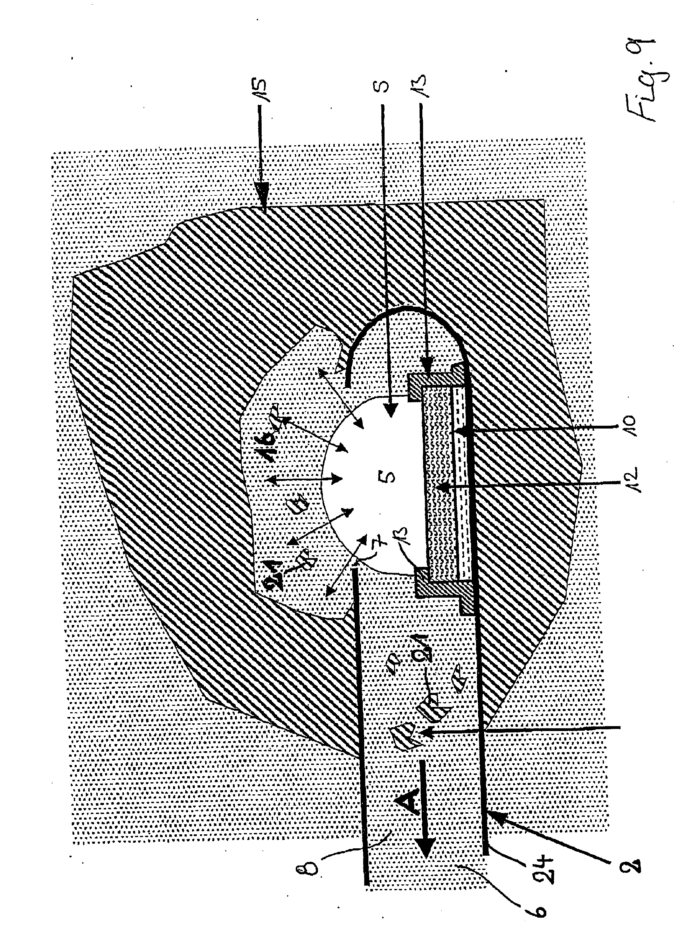

Fig. 9 zeigt die Anordnung eines Ablationswerkzeuges gemäß Fig. 3A innerhalb eines Applikatorstabes 2. Die Widerstandsheizschicht ist dabei mit ihrer Oberfläche auf einer der Seitenwandungen 24 des als Röhrchen ausgebildeten Applikatorstabes 2 angeordnet. Dieser gegenüber auf dem Umfang des Applikatorröhrchens 2 ist in der Wandung 24 eine Öffnung 7. Die auf der Oberfläche des Widerstandsheizelementes 12 erzeugte Dampfblase 5 kann nun durch diese Öffnung austreten und den Gegenstand 15 zerstören. In diesem Bereich werden Materialbruchstücke 21 abgetragen und in dem Gegenstand 15 eine Kavität 16 ausgebildet. Die Bruchstücke 21 können anschließend durch den als Absaugkanal 8 ausgebildeten Innenraum des Röhrchens 2 in Richtung des Pfeiles A abgesaugt werden.

Hier ist zu erwähnen, daß durch die lineare Bewegung des Applikatorröhrchens 2 ein Schnitt in das Material 15 eingebracht werden kann, so daß die erfindungsgemäße Vorrichtung sich auch zum Schneiden von Gegenständen eignet. Diese Vorrichtung kann auch in der Endoskopie eingesetzt werden.

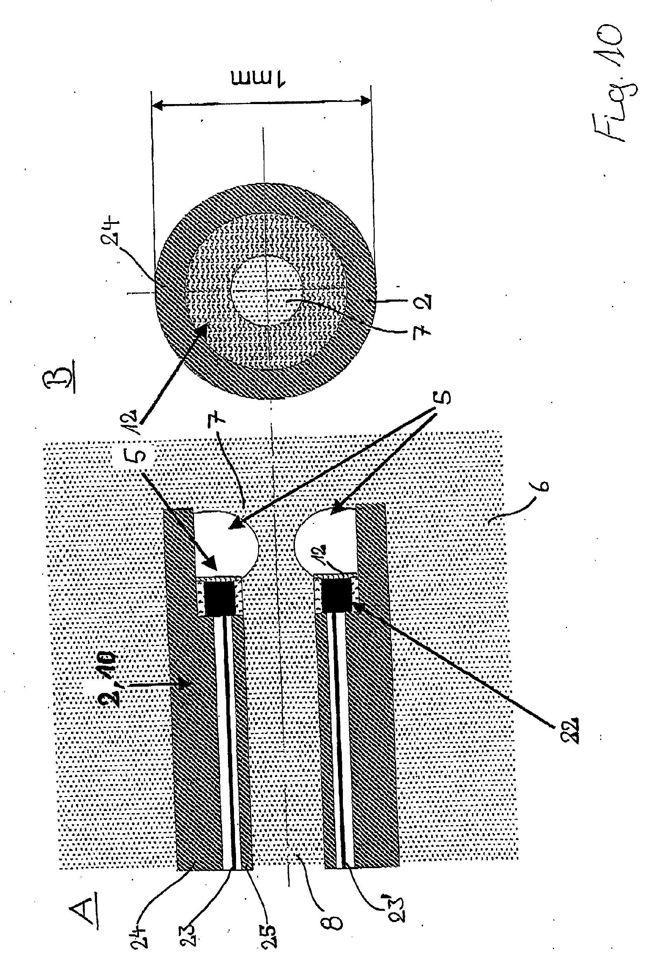

Fig. 10 zeigt ein weiteres Beispiel eines Ablationswerkzeuges in einem Applikatorröhrchen 2. Die Außenwandung 24 des Applikatorröhrchens 2 bildet zugleich das Substrat. Das Applikatorröhrchen besitzt, wie in Fig. 10A zu sehen ist, in axialer Richtung eine Öffnung 7. Fig. 10B zeigt eine Querschnittsansicht auf diese Öffnung 7. Wie zu erkennen ist, ist der Abstand von der Öffnung ringförmig die Öffnung umgeben eine Widerstandsheizschicht 12 angeordnet, die über einen Rückseitkontakt 22 und eine elektrische Zuleitung 23 und eine elektrische Ableitung 23' kontaktiert werden kann. Diese Leitungen 23, 23' verlaufen jeweils innerhalb von Öffnungen zwischen der Innenwandung 25 und der Außenwandung 24 des Applikatorröhrchens. Die Innenwandung 25 ist konzentrisch und schließt einen Hohlraum 8 als Absaugkanal ein.

Wird nun die Widerstandsheizschicht 12 mit einem elektrischen Strom beaufschlagt, so bildet sich eine radialsymmetrische Gasblase aus, die im Laufe des weiteren Heizens zusammenwächst und aus der Öffnung 7 ausgestoßen wird.

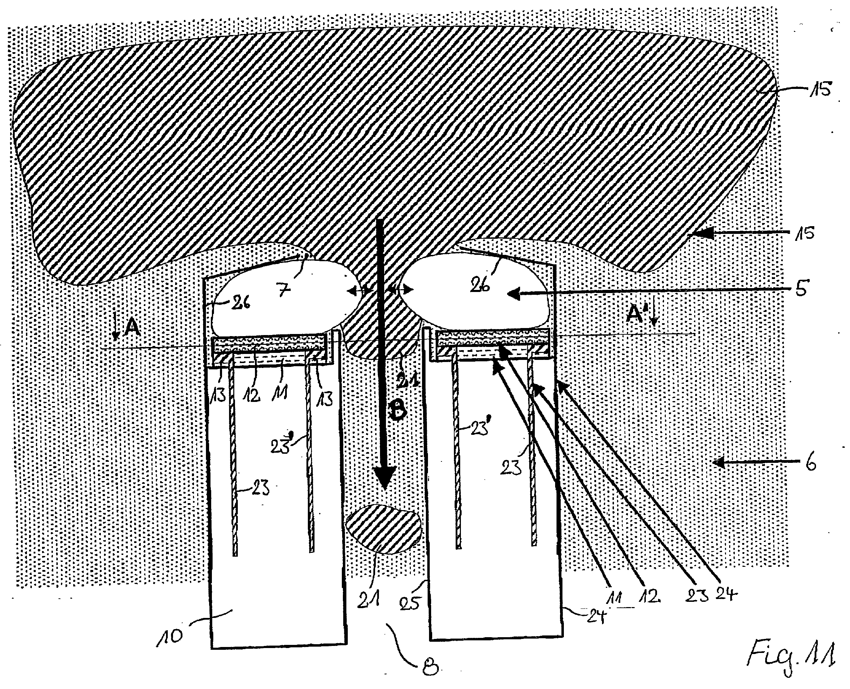

Fig. 11 zeigt eine Modifikation eines Ablationswerkzeuges, wie es in Fig. 10 dargestellt ist. Im Unterschied zu Fig. 10 erstreckt sich nun die Außenwandung 24 über die Widerstandsheizschicht 12 hinaus und winkelt dann nach innen in Richtung der Achse des Röhrchens 2 ab. Auf diese Weise wird die Widerstandsheizschicht 12 beabstandet überdeckt, wobei die Außenwandung 24 eine konzentrische Öffnung 7 zum Austritt der Gasblase freiläßt.

In Fig. 11 ist dargestellt, wie das erfindungsgemäße Applikationswerkzeug auch zum Abtrag eines flexiblen Materials 15 verwendet werden kann. Hierzu wird dieses Material 15 in die Öffnung 7 eingesaugt, wo die erzeugten Gasblasen 5 jeweils kleine Bruchstücke 21 abschnüren. Diese werden dann in Richtung des Pfeiles B durch den axial mittig verlaufenden Absaugkanal 8 abgesaugt.

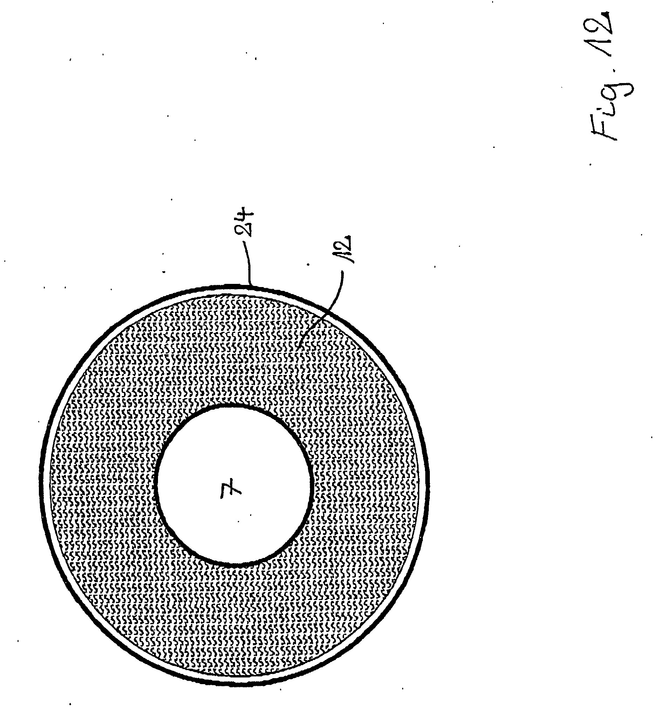

Fig. 12 zeigt einen Schnitt längs der Linie A-AN Fig. 11. Zu erkennen ist, daß wiederum die Widerstandsheizschicht 12 konzentrisch angeordnet ist und so eine radialsymmetrische Dampfblase ausbildet.

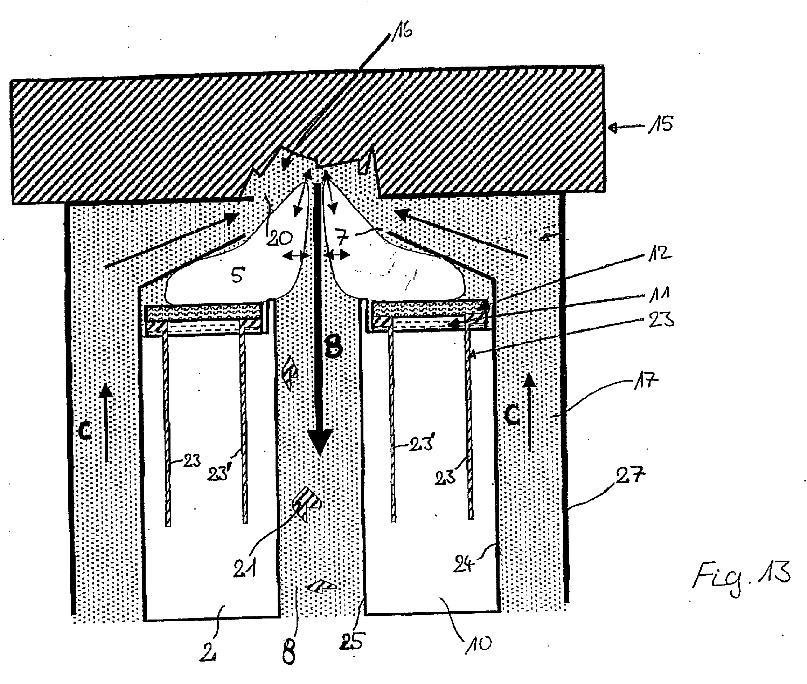

Fig. 13 zeigt ein weiteres erfindungsgemäßes Beispiel mit einem Ablationsinstrument, das demjenigen in Fig. 11 nachgebildet ist. Zusätzlich zu der Anordnung in Fig. 11 ist nunmehr das Röhrchen 2 in ein konzentrisch hierzu aber beabstandet angeordnetes Gehäuse 27 eingehäust. Diese Gehäuse 27 erstreckt sich längs der Außenwandung 24 des Röhrchens 2 und verengt sich in konzentrischer Weise nach innen oberhalb der Öffnung 7. Es bildet dadurch eine weitere Düsenöffnung 20 oberhalb der Öffnung 7 aus.

In dem Abstand zwischen der Außenwandung 24 und dem Gehäuse 27 ist ein Kapillarsystem 17 angeordnet, das der Zufuhr von Verdampfungsmedium zu dem Widerstandsheizelement 12 dient. Das Medium fließt in Richtung des Pfeiles C in den Bereich zwischen der Öffnung 7 und der Düsenöffnung 20. Beim Beheizen des Widerstandsheizelementes 12 wird wiederum eine konzentrische Gasblase 5 ausgebildet, die über die Düsenöffnung, die unmittelbar auf einem Gegenstand 15 aufgesetzt ist, auf den Gegenstand 15 einwirkt und diesen dort fragmentiert. Dabei wird eine Kavität 16 in dem Mediumstand 15 ausgebildet und die erzeugten Bruchstücke 21 des abgetragenen Materials werden über die Absaugöffnung 8 in Richtung des Pfeiles B abgesaugt.

Dadurch, daß bei diesem Beispiel das zu verdampfende Medium über den Kanal 17 zu der Oberfläche des Gegenstandes 15 zugeführt wird, ist es damit auch möglich, Festkörper zu fragmentieren, zu schneiden oder abzutragen.

## Patentansprüche

1. Ablationsinstrument zum Schneiden, Fragmentieren und/oder Abtragen von Material eines Gegenstandes
mit einem Trägersubstrat,
mindestens einer auf dem Substrat angeordneten Widerstandsheizschicht aus dotiertem oder graphitischem Diamant (DLC) sowie
mindestens einer elektrischen Zuleitung und mindestens einer elektrischen Ableitung, die beide an verschiedenen Stellen mit der Widerstandsheizschicht elektrisch kontaktiert sind.

2. Ablationsinstrument nach dem vorhergehenden Anspruch,
**dadurch gekennzeichnet, daß** das Substrat Silizium, Siliziumkarbid, Siliziumoxide wie SiO₂, Glas, refraktive Metalle oder Karbide hiervon, Saphir, Magnesiumoxid, Diamant, Graphit und/oder Germanium enthält oder daraus besteht.

3. Ablationsinstrument nach Anspruch 1,
**dadurch gekennzeichnet, daß** das Substrat zumindest bereichsweise eine Membran ist.

4. Ablationsinstrument nach einem der vorhergehenden Ansprüche,
**dadurch gekennzeichnet, daß** die Widerstandsheizschicht eine Fläche von weniger als 10 mm², bevorzugt weniger als 5 mm² aufweist.

5. Ablationsinstrument nach einem der vorhergehenden Ansprüche,
**dadurch gekennzeichnet, daß** die Widerstandsschicht eine Dotierstoffkonzentration größer oder gleich 5x10¹⁷ cm⁻³ aufweist.

6. Ablationsinstrument nach einem der vorhergehenden Ansprüche,
**dadurch gekennzeichnet, daß** die Widerstandsschicht mit Bor, Phosphor, Stickstoff, Lithium und/oder Schwefel dotiert ist.

7. Ablationsinstrument nach einem der vorhergehenden Ansprüche,
**dadurch gekennzeichnet, daß** die Widerstandsschicht eine thermische Leistungsdichte zwischen 0 und 1,5 GW/cm³ aufweist.

8. Ablationsinstrument nach einem der vorhergehenden Ansprüche,
**dadurch gekennzeichnet, daß** die Widerstandsschicht einen spezifischen Widerstand zwischen 1 µΩcm und 100 mΩcm aufweist.

9. Ablationsinstrument nach dem vorhergehenden Anspruch,
**dadurch gekennzeichnet, daß** zwischen dem Substrat und der Widerstandsheizschicht eine thermisch isolierende Schicht angeordnet ist.

10. Ablationsinstrument nach dem vorhergehenden Anspruch,
**dadurch gekennzeichnet, daß** die thermisch isolierende Schicht eine Wärmeleitfähigkeit von - unter 1 W·cm⁻¹·K⁻¹ bei 300 K aufweist.

11. Ablationsinstrument nach einem der beiden vorhergehenden Ansprüche,
**dadurch gekennzeichnet, daß** die thermisch isolierende Schicht aus einem amorphen Material - besteht.

12. Ablationsinstrument nach einem der drei vorhergehenden Ansprüche,
**dadurch gekennzeichnet, daß** die thermisch isolierende Schicht aus einem elektrischen Nichtleiter besteht.

13. Ablationsinstrument nach einem der vier vorhergehenden Ansprüche,
**dadurch gekennzeichnet, daß** das Material der thermisch isolierenden Schicht ausgewählt ist aus Siliziumoxiden, Siliziumnitriden, Siliziumoxinitriden und Aluminiumoxiden.

14. Ablationsinstrument nach einem der vorhergehenden Ansprüche,
**dadurch gekennzeichnet, daß** zwischen dem Substrat oder der thermisch isolierenden Schicht und der Widerstandsheizschicht mindestens eine Zwischenschicht angeordnet ist.

15. Ablationsinstrument nach dem vorhergehenden Anspruch,
**dadurch gekennzeichnet, daß** die Zwischenschicht aus intrinsischem Diamant besteht.

16. Ablationsinstrument nach einem der beiden vorhergehenden Ansprüche,
**dadurch gekennzeichnet, daß** die Zwischenschicht lateral strukturiert ist.

17. Ablationsinstrument nach einem der vorhergehenden Ansprüche,
**dadurch gekennzeichnet, daß** auf der Widerstandsschicht eine elektrisch isolierende Schicht angeordnet ist.

18. Ablationsinstrument nach einem der vorhergehenden Ansprüche,
**dadurch gekennzeichnet, daß** die
mit dem zu behandelnden Gegenstand kontaktierbare Oberfläche H-terminiert ist.

19. Ablationsinstrument nach einem der vorhergehenden Ansprüche,
**dadurch gekennzeichnet, daß** das Trägersubstrat an einem Ende eines Stabes angeordnet ist.

20. Ablationsinstrument nach dem vorhergehenden Anspruch,
**dadurch gekennzeichnet, daß** der Applikatorstab als Applikatorröhrchen ausgebildet ist, das im Bereich eines ersten Endes eine erste Öffnung aufweist und das Substrat in dem Hohlraum der ersten Öffnung benachbart derart angeordnet ist, daß die Oberfläche der Widerstandsheizschicht der ersten Öffnung zugewandt ist.

21. Ablationsinstrument nach dem vorhergehenden Anspruch,
**dadurch gekennzeichnet, daß** die erste Öffnung in axialer Richtung des Applikatorröhrchens oder seitlich auf dem Umfang des Applikatorröhrchens angeordnet ist.

22. Ablationsinstrument nach einem der drei vorhergehenden Ansprüche,
**dadurch gekennzeichnet, daß** das Substrat einen Außendurchmesser kleiner oder gleich dem Innendurchmesser des Applikatorröhrchens oder Applikatorstabes aufweist und senkrecht zur axialen richtung des Applikatorröhrchens oder Applikatorstabes angeordnet ist.

23. Ablationsinstrument nach einem der Ansprüche 19 bis 22,
**dadurch gekennzeichnet, daß** der Applikatorstab oder das Applikatorröhrchen eine Flüssigkeitszufuhr und/oder eine Flüssigkeitsabsaugung zu bzw. von der Oberfläche der Widerstandsheizschicht aufweist.

24. Ablationsinstrument nach dem vorhergehenden Anspruch,
**dadurch gekennzeichnet, daß** die Flüssigkeitszufuhr und/oder die Flüssigkeitsabsaugung als Durchgangsöffnung durch das Substrat, die Zwischenschicht, die thermisch isolierende Schicht und die Widerstandsschicht durchtreten.

25. Ablationsinstrument nach einem der Ansprüche 19 bis 24,
**dadurch gekennzeichnet, daß** in das Applikatorröhrchen ein weiteres Innenröhrchen eingefügt - ist, das den Innenraum des Applikatorröhrchens in zwei axial sich erstreckende Kompartimente aufteilt.

26. Ablationsinstrument nach einem der Ansprüche 19 bis 24,
**dadurch gekennzeichnet, daß** in das Applikatorröhrchen ein weiteres Innenröhrchen eingefügt ist, das den Innenraum des Applikatorröhrchens in zwei konzentrisch sich erstreckende Kompartimente aufteilt.

27. Ablationsinstrument nach einem der Ansprüche 19 bis 24,
**dadurch gekennzeichnet, daß** in das Applikatorröhrchen ein weiteres Innenröhrchen eingefügt ist, das den Innenraum des Applikatorröhrchens in zwei parallel zueinander verlaufende Kompartimente aufteilt.

28. Ablationsinstrument nach dem vorhergehenden Anspruch,
**dadurch gekennzeichnet, daß** die beiden Kompartimente Teil der Flüssigkeitszufuhr und/oder Flüssigkeitsabsaugung sind.

29. Ablationsinstrument nach dem vorhergehenden Anspruch,
dadurch gekennzeichnt, daß das Substrat an dem dem ersten Ende des Applikatorröhrchens zugewandten Ende des Innenröhrchens angeordnet ist.

30. Ablationsinstrument nach einem der vorhergehenden Ansprüche,
**dadurch gekennzeichnet, daß** über der Widerstandsheizschicht eine Düsenplatte angeordnet ist, die oberhalb der Widerstandsheizschicht eine Düsenöffnung aufweist.

31. Ablationsinstrument nach einem der vorhergehenden Ansprüche,
**dadurch gekennzeichnet, daß** über der Widerstandsheizschicht eine Platte angeordnet ist, die Kanäle zur Zufuhr und/oder Absaugung von Flüssigkeit zu der Oberfläche der Heizschicht aufweist.

32. Ablationsinstrument nach dem vorhergehenden Anspruch,
**dadurch gekennzeichnet, daß** die Kanäle als Kapillaren ausgebildet sind.

33. Ablationsinstrument nach einem der vorhergehenden Ansprüche,
**dadurch gekennzeichnet, daß** die elektrische Ableitung mindestens eine Kontaktmaterialschicht, eine darauf angeordnete Diffusionsbarriere und eine auf der Diffusionsbarriere angeordnete metallische Deckschicht aufweist.

34. Ablationsinstrument nach dem vorhergehenden Anspruch,
**dadurch gekennzeichnet, daß** die Kontaktmaterialschicht aus amorphem Silizium besteht.

35. Ablationsinstrument nach einem der beiden vorhergehenden Ansprüche,
**dadurch gekennzeichnet, daß** die Diffusionsbarriere aus N/W, Ti/Au, C/Au, WC/Au, Ti/Pt/Au und/oder leitfähigem diamantartigen Kohlenstoff (DLC) besteht.

36. Ablationsinstrument nach einem der vorhergehenden Ansprüche,
**dadurch gekennzeichnet, daß** im Bereich der Widerstandsheizschicht ein Temperatursensor angeordnet ist.

37. Ablationsinstrument nach dem vorhergehenden Anspruch,
**dadurch gekennzeichnet, daß** die Widerstandsschicht selbst als Temperatursensor ausgebildet ist.

38. Ablationsverfahren zum Schneiden, Fragmentieren von Material oder Abtragen von Material von einer Oberfläche eines Gegenstandes, wobei eine Widerstandsheizschicht aus Diamant in unmittelbarem Kontakt mit dem oder in die Nähe des zu behandelnden Materials gebracht wird und gegebenenfalls eine Flüssigkeit zwischen die Diamantschicht und das zu behandelnde Material eingebracht wird;
die Diamantschicht mit zwei elektrischen Leitern kontaktiert und über die elektrischen Leiter zur Aufheizung mit einem elektrischen Strom derart beaufschlagt wird, daß zwischen der Diamantschicht und dem Gegenstand befindliche Flüssigkeit und/oder das zu behandelnde Material an der Kontaktstelle zu der Widerstandsschicht Dampfblasen ausbildet.

39. Ablationsverfahren nach dem vorhergehenden Anspruch,
**dadurch gekennzeichnet, daß** die Widerstandsheizschicht in Kontakt mit oder in die Nähe der Oberfläche des zu behandelnden Materials aufgebracht oder in das zu behandelnde Material eingebracht wird.

40. Ablationsverfahren nach einem der beiden vorhergehenden Ansprüche,
**dadurch gekennzeichnet, daß** die Flüssigkeit und/oder das aus dem zu behandelnden Gegenstand abgetragene Material abgesaugt wird.

41. Ablationsverfahren nach einem der drei vorhergehenden Ansprüche,
**dadurch gekennzeichnet, daß** ein Ablationsinstrument nach einem der Ansprüche 1 bis 32 verwendet wird.

42. Verwendung eines Ablationsinstrumentes oder eines Ablationsverfahrens nach einem der vorhergehenden Ansprüche zur Materialbehandlung und -formung, zum Schneiden, zum Fragmentieren und/oder Abtragen von Material, insbesondere in der Medider Medizin , insbesondere in der Chirurgie, der Augenchirugie, insbesondere zur Kataraktextration oder Vitrektomie.
